# EUROPÄISCHE PATENTSCHRIFT

(11) **EP 1 007 018 B1**
(45) Veröffentlichungstag und Bekanntmachung des Hinweises auf die Patenterteilung: **08.10.2003**
(21) Anmeldenummer: 98908079.1
(22) Anmeldetag: 11.02.1998
(51) Int. Cl.: A61K 31/122, A61P 27/16

(54) **VERWENDUNG VON 2,3-DIMETHOXY-5-METHYL-6-DECAPRENYL-1,4-BENZOCHINON INDER BEHANDLUNG VON TINNITUS**
THE USE OF 2,3-DIMETHOXY-5-METHYL-6-DECAPRENYL-1,4-BENZOQUINONE IN THE TREATMENT OF TINNITUS
UTILISATION DE 2,3-DIMETHOXY-5-METHYLE-6-DECAPRENYLE-1,4-BENZOQUINONE DANS LE TRAITEMENT DE TINNITUS

(30) Priorität: 12.02.1997 DE 19705232
(43) Veröffentlichungstag der Anmeldung: 14.06.2000
(73) Patentinhaber: MSE Pharmazeutika GmbH, 61348 Bad Homburg (DE)
(72) Erfinder: ENZMANN, Franz, D-61348 Bad Homburg (DE); LACHMANN, Burkhard, NL-3065 BC Rotterdam (NL)
(74) Vertreter: Werner, Hans-Karsten, Dr.Dipl.-Chem.
(86) Internationale Anmeldenummer: EP9800743
(87) Internationale Veröffentlichungsnummer: WO98035658

(56) Entgegenhaltungen:
- GB-A- 2 184 355
- US-A- 4 824 669
- US-A- 5 451 569
- PATENT ABSTRACTS OF JAPAN vol. 012, no. 248 (C-511), 13.Juli 1988 & JP 63 036789 A (MITSUBISHI GAS CHEM CO INC), 17.Februar 1988,
- JAMES E F REYNOLDS: "MARTINDALE The Extra Pharmacopoeia Thirty-first Edition" 1996 , ROYAL PHARMACEUTICAL SOCIETY , LONDON, GB XP002078664 Page 1764: Ubidecarenone

## Beschreibung

2,3-Dimethoxy-5-methyl-6-decaprenyl-1,4-benzochinon ist auch bekannt unter der Bezeichnung Coenzym Q10. Die Substanz spielt eine Rolle in der Atmungskette und ist obendrein ein Antioxidanz, welches in der Lage ist, Radikale, die insbesondere von Vitaminen weitergegeben werden, abzufangen und unschädlich zu machen. Q-10 bestimmt außerdem die Elastizität und die Dynamik der Zellmembranen. Es wird daher als Monopräparat und in Kombination mit anderen Wirkstoffen zur oralen Einnahme empfohlen. Zur Hautpflege wird es darüber hinaus in Form einer Liposomencreme angeboten, welche es dem Wirkstoff gestattet, durch die Hornschichtbarrieren einzudringen und sich dann in den verschiedenen Schichten der Haut anzureichern. Die bisher verwendete Liposomcreme wird hergestellt auf Basis von Lecithinen und bildet eine Lipiddoppelschicht um einen wäßrigen Innenraum. Q-10 lagert sich dabei innerhalb der Membran an.

Es wurde jetzt gefunden, daß diese Substanz in weit größerem Umfang als bisher bekannt und vorherzusehen war, geeignet ist zur oralen Behandlung von Tinnitus.

Zur oralen Anwendung kann es eingesetzt werden in Form von Pulver in Hartgelatinekapseln oder in öliger Suspension in Weichgelatinekapseln. Die Anmelderin vertreibt ein derartiges Monopräparat mit 30 mg des Wirkstoffes und auf ärztliche Anforderung mit 120 mg Q-10.

Der Pulmonary Surfactant ist ein aus den Lungen isolierbarer Komplex von speziellen Phospholipiden, neutralen Lipiden und Surfactant Proteinen, die miteinander eine einschichtige Barriere zwischen der Luft und der flüssigen Oberfläche der Lunge ausbilden. Der Pulmonary Surfactant wird in den Alveolaren Typ II Zellen gebildet und von dort in den Alveolarzwischenraum abgegeben. Der Pulmonary Surfactant kann auch aus den Komponenten rekombiniert werden.

Der Pulmonary Surfactant ist bisher nur eingesetzt worden zur Instillation bei Erkrankungen oder Mangelerscheinungen der Lunge.

Andere Anwendungen sind bisher nicht in Erwägung gezogen worden: Es wurde nun gefunden, daß Pulmonary Surfactant unerwarteterweise in die äußere Haut und die Schleimhaut des Magen-Darmbereiches, des Mund- und Vaginalbereiches einzudringen vermag, und zwar allein oder in Zusammenwirkung mit Liposomen.

Dabei spielt es eine untergeordnete Rolle, ob hochgereinigte oder weniger gereinigte Pulmonary Surfactant-Präparationen aller möglichen Spezies oder rekombinierter Pulmonary Surfactant herangezogen werden (Schwein, Rind, Schaf, etc). Weniger gereinigte Präparationen haben den Vorteil, daß sie sich kostengünstig produzieren lassen.

Pulmonary Surfactant ist somit geeignet, die Wirksamkeit von 2,3-Dimethoxy-5-methyl-6-decaprenyl-1,4-benzochinon zu verbessern und den Wirkungseintritt zu beschleunigen. Obwohl physiologischerweise Pulmonary Surfactant aus dem Lungengewebe abgegeben wird, vermag Pulmonary Surfactant überraschenderweise die Gewebsaufnahme von Pulmonary Surfactant und Q-10 zu steigern.

Völlig neu sind die Wirksamkeit von Q-10 allein und in Kombination mit Pulmonary Surfactant bei der Behandlung von Tinnitus.

## Patentansprüche

1. Verwendung von 2,3-Dimethoxy-5-methyl-6-decaprenyl-1,4-benzochinon zur Herstellung von Arzneimitteln zur oralen Behandlung von Tinnitus.

2. Verwendung nach Anspruch 1, **dadurch gekennzeichnet, daß** die Zubereitungen zusätzlich eine wirksame Menge des Pulmonary Surfactant enthalten.

3. Verwendung gemäß Anspruch 2, **dadurch gekennzeichnet, daß** der Pulmonary Surfactant als Rohextrakt eingesetzt wird.

4. Verwendung nach Anspruch 2 oder 3, **dadurch gekennzeichnet, daß** die Zubereitungen Liposome enthalten.

## Claims

1. Use of 2,3-dimethoxy-5-methyl-6-decaprenyl-1,4-benzoquinone for preparing medicaments for the oral treatment of tinnitus.

2. The use according to claim 1, **characterized in that** the formulations additionally contain an effective amount of pulmonary surfactant.

3. The use according to claim 2, **characterized in that** said pulmonary surfactant is employed as a raw extract.

4. The use according to claim 2 or 3, **characterized in that** the formulations contains liposomes.

## Revendications

1. Utilisation de la 2,3-diméthoxy-5-méthyl-6-décaprényl-1,4-benzoquinone pour préparer des médicaments destinés au traitement par voie orale des bourdonnements.

2. Utilisation selon la revendication 1, **caractérisée en ce que** les préparations contiennent en plus une quantité active de surfactant pulmonaire.

3. Utilisation selon la revendication 2, **caractérisée en ce qu'**on utilise le surfactant pulmonaire sous forme d'extrait brut.

4. Utilisation selon la revendication 2 ou 3, **caractérisée en ce que** les préparations contiennent des liposomes.
